# EUROPÄISCHE PATENTANMELDUNG

(11) **EP 3 834 937 A1**
(43) Veröffentlichungstag der Anmeldung: **16.06.2021**
(21) Anmeldenummer: 19405020.9
(22) Anmeldetag: 10.12.2019
(51) Int. Cl.: B01L 1/04, A61L 2/00, B08B 15/02

(54) **AUFBAU EINES CONTAINMENTS MIT EINER ASEPTISCHEN ARBEITSKAMMER**

(71) Anmelder: SKAN AG, 4123 Allschwil (CH)
(72) Erfinder: Lehmann, Frank Martin, CH-4102 Binningen (CH); Scholler, Yves, F-68220 Hegenheim (FR); Sommerhalder, Matthias, CH-4112 Bättwil (CH)
(74) Vertreter: Ullrich, Gerhard

(57) **Zusammenfassung**

Gegenstand ist der Aufbau eines Containments (**1**) mit einer aseptischen Arbeitskammer (**11**) und zugehöriger Dekontaminationsanordnung (**6**). Die Arbeitskammer (**14**) ist unten von einem Boden (**12**) begrenzt, und oberhalb der Arbeitskammer (**14**) befindet sich eine Umluftzone (**15**), in der ein Umluftfilter (**2,2'**) mit einem Umluftventilator (**3**) angeordnet ist. Die Umluftzone (**15**) und die Arbeitskammer (**14**) sind durch ein Gehäuse (**10**) nach aussen abgegrenzt. Aus der Arbeitskammer (**14**) führt zumindest ein erster Rückluftkanal (**16**) in die Umluftzone (**15**). Ein Rückluftfilter (**5,5'**) ist strömungstechnisch mit dem Rückluftkanal (**16**) verbunden, nämlich der Umluftzone (**15**) zugewandt angeordnet. Der Rückluftfilter (**5,5'**) kann an der Mündung des Rückluftkanals (**16**) in die Umluftzone (**15**) oder im Rückluftkanal (**16**), rückversetzt von der Mündung, angeordnet sein. Rückluftfilter (**5,5'**) bzw. Umluftfilter (**2,2'**) haben die Gestalt eines Plattenfilters oder einer auswechselbaren Filterpatrone (**5',2'**) oder eines ersetzbaren Filtereinsatzes. Hierbei kann ein einzelner Rückluftfilter (**5,5'**) oder eine Serie von zusammenwirkenden Rückluftfiltern (**5,5'**) bzw. ein einzelner Umluftfilter (**2,2'**) oder eine Serie von zusammenwirkenden Umluftfiltern (**2,2'**) installiert sein. Containments (**1**), in denen grössere Luftvolumina zu verarbeiten sind, haben zusätzlich einen zweiten Rückluftkanal (**17**), an oder in dem ebenfalls ein Rückluftfilter (**5,5'**) eingebaut ist.

## Beschreibung

### Anwendungsgebiet der Erfindung

Die vorliegende Erfindung hat den Aufbau eines Containments mit einer aseptischen Arbeitskammer und einer zugeordneten Dekontaminationsanordnung zum Gegenstand. Die Arbeitskammer ist unten von einem Boden begrenzt, und oberhalb der Arbeitskammer befindet sich eine Umluftzone, in der ein Umluftfilter mit einem Umluftventilator angeordnet ist. Die Umluftzone und die Arbeitskammer sind durch ein Gehäuse nach aussen abgegrenzt. Aus der Arbeitskammer führt zumindest ein erster Rückluftkanal in die Umluftzone. Ein Rückluftfilter ist strömungstechnisch mit dem Rückluftkanal verbunden. Solche Anlagen dienen der Behandlung von sterilen Gütern, bei der keine verunreinigenden Partikel von aussen an das Behandlungsgut gelangen dürfen. Als Containments gelten insbesondere Isolatoren, z.B. für die pharmazeutisch-chemische Industrie. Der Begriff umfasst ferner alle Typen von RABS (Restricted Access Barrier System).

### Stand der Technik

In der EP 3 170 545 A1 der Anmelderin ist der beispielhafte Aufbau eines Containments gezeigt. Auf der Frontseite sind in einer transparenten Scheibe Handschuheingriffe eingesetzt, so dass die Bedienperson Einblick und mit den Handschuhen Zugriff in die Arbeitskammer hat. Die in die Arbeitskammer eingebrachte laminare Luftströmung wird über einen Rückluftkanal, von Filtereinheiten gereinigt, in die oberhalb der Arbeitskammer gelegene Umluftzone geleitet. Die Filtereinheiten, z.B. in Gestalt von Filterpatronen, sind am erweiterten Eintritt in den Rückluftkanal eingesetzt und erlauben so ein vorteilhaftes Handling beim Filterwechsel. Bei grösserer Dimensionierung des erweiterten Eintritts könnte sich dadurch aber für die Bedienperson die Ergonomie am Arbeitsplatz vor dem Containment nicht ganz optimal gestalten.

Die EP 2 666 532 B1 offenbart einen etwas anderen Aufbau eines Containments. Hier sind zur Rückführung der in die Arbeitskammer eingebrachten laminaren Luftströmung in die über der Arbeitskammer angeordneten Umluftzone zwei Rückluftkanäle vorgesehen, an deren Einströmöffnung jeweils eine Filtereinheit installiert ist, die auf dem Boden der Arbeitskammer ruhen. Damit wird jedoch der in der Arbeitskammer für darin positionierte Apparaturen verfügbare Raum eingeengt und die Bedienperson muss mit den Arbeitshandschuhen die Filtereinheit übergreifen, quasi weiter vorstrecken, um an die beabsichtigte Stelle, z.B. an einer Prozessmaschine, zu gelangen.

### Aufgabe der Erfindung

Ausgehend vom vorbekannten Stand der Technik liegt der Erfindung die Aufgabe zugrunde, den strukturellen Aufbau eines Containments mit einer aseptischen Arbeitskammer für die am Containment tätige Bedienperson ergonomisch optimaler zu gestalten und dabei den in der Arbeitskammer verfügbaren Raum bestmöglich für den Betrieb und die Aufstellung von Apparaturen zu nutzen. Schliesslich soll das reinraumtechnische Konzept zu effizienten Betriebs- und Anlagenkosten führen.

### Übersicht über die Erfindung

Gegenstand ist der Aufbau eines Containments mit einer aseptischen Arbeitskammer und zugehöriger Dekontaminationsanordnung. Die Arbeitskammer ist unten von einem Boden begrenzt, und oberhalb der Arbeitskammer befindet sich eine Umluftzone, in der ein Umluftfilter mit einem Umluftventilator angeordnet ist. Die Umluftzone und die Arbeitskammer sind durch ein Gehäuse nach aussen abgegrenzt. Aus der Arbeitskammer führt zumindest ein erster Rückluftkanal in die Umluftzone. Ein Rückluftfilter ist strömungstechnisch mit dem Rückluftkanal verbunden, nämlich der Umluftzone zugewandt angeordnet.

Nachfolgend werden spezielle Ausführungsformen der Erfindung definiert: Der Rückluftfilter kann an der Mündung des Rückluftkanals in die Umluftzone oder im Rückluftkanal, rückversetzt von der Mündung, angeordnet sein. Die den Rückluftfilter aufnehmende Mündung des Rückluftkanals kann als die Umluftzone erweiternde oder in die Umluftzone hineinragende Auskragung ausgebildet sein oder geht ohne Auskragung, quasi mit linearem Querschnitt, in die Umluftzone über. Der Rückluftfilter bzw. der Umluftfilter hat die Gestalt eines Plattenfilters oder einer auswechselbaren Filterpatrone oder eines ersetzbaren Filtereinsatzes. Hierbei kann man einen einzelnen Rückluftfilter oder eine Serie von zusammenwirkenden Rückluftfiltern vorsehen bzw. einen einzelnen Umluftfilter oder eine Serie von zusammenwirkenden Umluftfiltern installieren.

Der zumindest erste Rückluftkanal weist eine transparente Frontscheibe und eine dazu beabstandet angeordnete transparente Kanalscheibe als Bestandteile auf und besitzt eine Einströmöffnung, die sich oberhalb und nahe des Bodens befindet.

Zwischen der Umluftzone und der Arbeitskammer erstreckt sich ein Luftleitelement, das zur Erzeugung einer in der Arbeitskammer abwärts fliessenden, gleichgerichteten Verdrängungsströmung (Laminar Flow) dient.

Die Dekontaminationsanordnung weist einen in der Umluftzone positionierten Verdampfer oder zumindest eine Vernebelungsdüse auf. Dem Verdampfer bzw. der Vernebelungsdüse wird über eine Stoffleitung Dekontaminationsmittel mittels einer Pumpe zugeführt, wobei die Vernebelungsdüse das Dekontaminationsmittel mit Druckluft als Aerosol zerstäubt.

Die zumindest eine Vernebelungsdüse ist gerichtet in:
- die Arbeitskammer; und/oder
- einen Zwischenraum, der sich zwischen der Umluftzone und dem Luftleitelement befindet; und/oder
- den zumindest ersten Rückluftkanal.

Beim Betrieb des Containments im Dekontaminationsmodus mit dem Verdampfer als Teil der Dekontaminationsanordnung wird der Umluftventilator mit verminderter Leistung, z.B. 50%, gesteuert.

Je nach Grösse und Bestimmung der Arbeitskammer kann eine Serie von in die Arbeitskammer gerichteten Vernebelungsdüsen vorgesehen sein. Beim Betrieb des Containments im Dekontaminationsmodus mit den Vernebelungsdüsen in der Dekontaminationsanordnung ist der Umluftventilator zumindest soweit heruntergeregelt, dass kein Volumenstrom gefördert wird. Vorteilhaft sind die Vernebelungsdüsen zueinander so versetzt angeordnet, dass das als Dekontaminationsmittel eingebrachte Aerosol in der Arbeitskammer zirkuliert.

In weiterer Komplettierung des Aufbaus ist eine Waschdüse gerichtet in:
- die Arbeitskammer; und/oder
- den Zwischenraum, der sich zwischen der Umluftzone und dem Luftleitelement befindet; und/oder
- den zumindest ersten Rückluftkanal, nämlich entgegen der Strömungsrichtung vor dem Rückluftfilter.
Die in den Rückluftkanal und/oder in den Zwischenraum gerichteten Waschdüsen sind vorzugsweise fest installiert, während die in die Arbeitskammer gerichtete Waschdüse alternativ an einer Handpistole sitzen kann.

Insbesondere Containments, mit spezieller Bestimmung oder in denen grössere Luftvolumina zu verarbeiten sind, haben zusätzlich einen zweiten Rückluftkanal, der einen Wandabschnitt des Gehäuses und eine dazu beabstandete Kanalwandung aufweist und eine Einströmöffnung besitzt, die sich wiederum oberhalb und nahe des Bodens befindet. Auch der zum zweiten Rückluftkanal zugehörige Rückluftfilter ist der Umluftzone zugewandt angeordnet, nämlich an der Mündung des zweiten Rückluftkanals in die Umluftzone oder direkt im zweiten Rückluftkanal. Die den Rückluftfilter aufnehmende Mündung des zweiten Rückluftkanals kann gleichfalls als die Umluftzone erweiternde oder in die Umluftzone hineinragende Auskragung ausgebildet sein, oder geht ohne Auskragung, quasi mit linearem Querschnitt, in die Umluftzone über. Wie für den ersten Rückluftkanal, hat auch der Rückluftfilter für den zweiten Rückluftkanal die Gestalt eines Plattenfilters oder einer auswechselbaren Filterpatrone oder eines ersetzbaren Filtereinsatzes.

Auch bei Vorhandensein von zwei Rückluftkanälen kann der Umluftfilter als Plattenfilter oder auswechselbare Filterpatrone oder ersetzbarer Filtereinsatz beschaffen sein. Dabei können ein einzelner Rückluftfilter oder eine Serie von zusammenwirkenden Rückluftfiltern bzw. ein einzelner Umluftfilter oder eine Serie von zusammenwirkenden Umluftfiltern installiert sein. Wie für den ersten Rückluftkanal, lassen sich auch für den zweiten Rückluftkanal eine Vernebelungsdüse und eine Waschdüse installieren.

### Kurzbeschreibung der beigefügten Zeichnungen

Dargestellt sind der erfindungsgemässe Aufbau eines Containments mit einer aseptischen Arbeitskammer in verschiedenen Ausführungsformen, jeweils als Prinzipdarstellung, nämlich:
- Figur 1 -: eine *erste Ausführungsform* mit einem Rückluftkanal mit einer in die Umluftzone hineinragenden Auskragung, darin installiertem Rückluftfil-ter, einem Umluftfilter und einem Verdampfer als Bestandteil der De-kontaminationsanord nung;
- Figur 2 -: eine *zweite Ausführungsform* im Aufbau gemäss Figur 1 mit zwei Rück-luftkanälen;
- Figur 3 -: eine *dritte Ausführungsform* mit einem Rückluftkanal mit einer die Um-luftzone erweiternden Auskragung, darin installiertem Rückluftfilter, einem Umluftfilter und Verneblungsdüsen als Bestandteil der Dekonta-minationsanordnung ;
- Figur 4 -: eine *vierte Ausführungsform* mit zwei Rückluftkanälen, jeweils mit einer in die Umluftzone hineinragenden Auskragung, darin installiertem Rück-luftfilter, einem Umluftfilter und Verneblungsdüsen als Bestandteil der Dekontaminationsanordnung;
- Figur 5 -: eine *fünfte Ausführungsform* im Aufbau gemäss Figur 4 mit zwei Rück-luftkanälen, jeweils mit einer die Umluftzone erweiternden Auskragung, darin installiertem Rückluftfilter und Verneblungsdüsen als Bestandteil der Dekontaminationsanordnung;
- Figur 6 -: eine *sechste Ausführungsform* im Aufbau gemäss Figur 5 mit je einer auswechselbaren Filterpatrone als Rückluftfilter; und
- Figur 7 -: eine *siebente Ausführungsform* mit zwei Rückluftkanälen mit je einer auswechselbaren Filterpatrone als Rückluftfilter, in Serie angeordneten auswechselbaren Filterpatronen als Umluftfilter und Verneblungsdüsen als Bestandteil der Dekontaminationsanordnung.

### Ausführunasbeispiel

Mit Bezug auf die beiliegenden Zeichnungen erfolgt nachstehend die detaillierte Beschreibung des erfindungsgemässen Aufbaus eines Containments mit einer aseptischen Arbeitskammer in verschiedenen beispielhaften Ausführungsformen.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

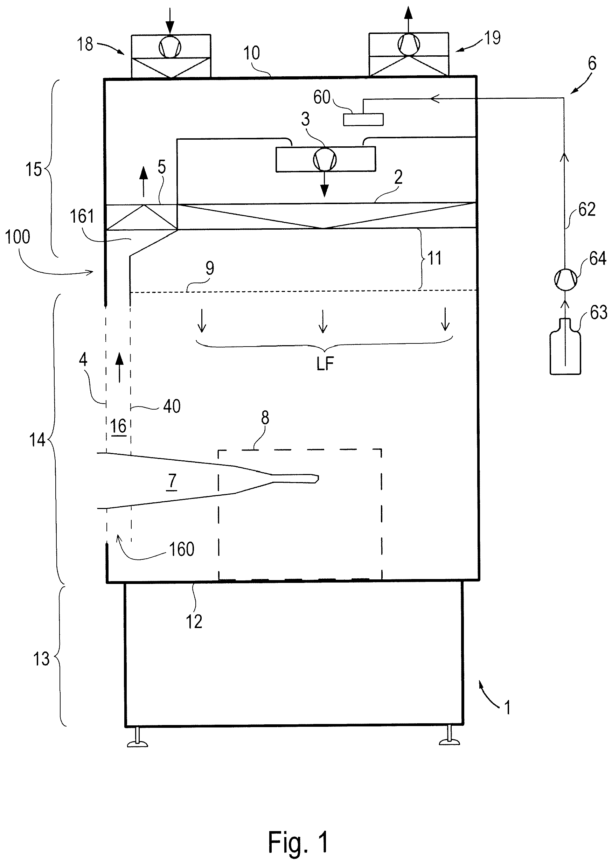

### Figur 1 - Erste Ausführungsform

Stehend und von unten aufwärts betrachtet, ist das Containment **1** in den Unterbau **13**, die darüber liegende Arbeitskammer **14**, den darüber liegenden Zwischenraum **11** sowie die zuoberst angeordnete Umluftzone **15** strukturiert. Arbeitskammer **13**, Zwischenraum **11** und Umluftzone **15** sind durch das Gehäuse **10** nach aussen abgegrenzt. Unten endet die Arbeitskammer **14** mit dem Boden **12** und oben mit dem als Laminarisator ausgebildeten Luftleitelement **9** - hier ein sogenannter CG-Verteiler in Gestalt eines netzartigen Sterilluftverteilers. An die Umluftzone **15** sind eine Zulufteinheit **18** sowie die Ablufteinheit **19** angeschlossen, jeweils aus einem Ventilator und einem Filter bestehend. Über die Zulufteinheit **18** wird der im Betriebsmodus im Containment **1** zirkulierenden Luft der Anteil aufbereiteter Frischluft in die Umluftzone **15** eingespeist, welcher aus der Umluftzone **15** über die Ablufteinheit **19** abgeführt wird. Innerhalb der Umluftzone **15** sind der Umluftventilator **3** und der Umluftfilter **2** - hier in Gestalt eines Plattenfilters - angeordnet. Im Betriebsmodus saugt der Umluftventilator **3** Luft aus der Umluftzone **15** an und fördert diese durch den Umluftfilter **2** in den Zwischenraum **11** und anschliessend durch das darunter aufgespannte Luftleitelement **9** als abwärts in der Arbeitskammer **14**, in Richtung Boden **12**, fliessende gleichgerichtete Verdrängungsströmung, vereinfacht Laminar Flow LF genannt. Zumeist ist in der Arbeitskammer **14**, in der Regel auf dem Boden **12** aufgestellt, zumindest eine Prozessmaschine **8**, z.B. eine Abfüllmaschine, installiert.

Das Containment **1** besitzt zumindest einen ersten Rückluftkanal **16**, welcher auf der Frontseite **100** einerseits anteilig vom Gehäuse **10** und der transparenten Frontscheibe **4** und andererseits von der transparenten Kanalscheibe **40** ummantelt ist; Frontscheibe **4** und Kanalscheibe **40** zusammen werden in der Branche oft als Doppelscheibe bezeichnet. Auf der Frontseite **100** ist üblicherweise ein Paar von Arbeitshandschuhen **7** angebracht, das einem Bediener den geschützten Zugriff in die Arbeitskammer **14** ermöglicht. Der Rückluftkanal **16** erstreckt sich von der Nähe des Bodens **12**, weist hier die Einströmöffnung **160** auf, bis in die Umluftzone **15**. An der Mündung des Rückluftkanals **16** in die Umluftzone **15,** nämlich im hiesigen Beispiel in einer in die Umluftzone **15** hineinragenden Auskragung **161**, ist ein Rückluftfilter **5** eingebaut, derzeit in Gestalt eines HEPA-Plattenfilters (High Efficiency Particulate Air Filter). Durch Förderwirkung des Umluftventilators **3** zirkuliert im Containment **1** die Luft im Betriebsmodus aus der Umluftzone **15** über die Arbeitskammer **14** und durch den Rückluftkanal **16** zurück in die Umluftzone **15** - dies bei anteiligem Ersatz von über die Ablufteinheit **19** abgeführter Luft durch mittels der Zulufteinheit **18** eingebrachter, aufbereiteter Frischluft. Hierbei hat der Rückluftfilter **5** eine zweifache Funktion. Erstens werden eventuelle aus der Arbeitskammer **14** mit dem Rückluftstrom mitgeführte unreine Partikel im Rückluftfilter **5** abgeschieden. Zweitens können keine eventuellen unreinen Partikel aus der Umluftzone **15** - zumal gegen den zirkulierenden Luftstrom - durch den auch als Rückflussverhinderer wirkenden Rückluftfilter **5** über den Rückluftkanal **16** bis in die Arbeitskammer **14** gelangen.

Für die Durchführung einer Dekontaminationsphase ist das Containment **1** mit einer Dekontaminationsanordnung **6** ausgestattet. In der illustrierten Ausführungsform ist ein in der Umluftzone **15** positionierter Verdampfer **60** vorgesehen, dem mittels einer Pumpe **64** aus einem Vorratsbehälter **63** über eine Stoffleitung **62** Dekontaminationsmittel, vorzugsweise eine H₂O₂-Lösung, zugeführt wird. Im Dekontaminationsmodus wird der Umluftventilator **3** mit verminderter Leistung, z.B. 50%, gesteuert.

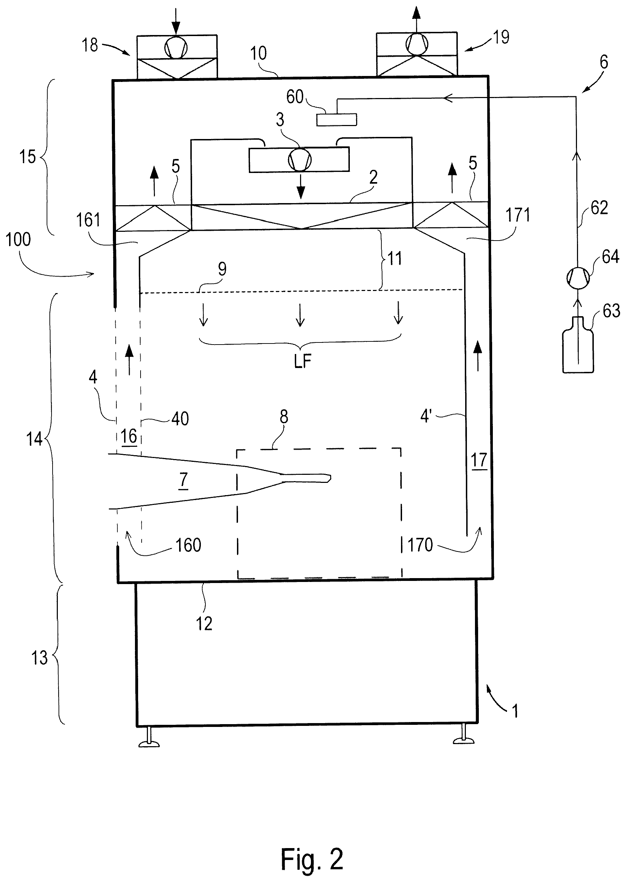

### Figur 2 - Zweite Ausführungsform

Der Unterschied zur ersten Ausführungsform gemäss Figur 1 besteht nur darin, dass jetzt zusätzlich ein zweiter Rückluftkanal **17** vorgesehen ist, der einen Wandabschnitt des Gehäuses **10** und eine dazu beabstandete Kanalwandung **4'** aufweist und die Einströmöffnung **170** besitzt, die sich wiederum oberhalb und nahe des Bodens **12** befindet. Dabei ist auch an der Mündung des zweiten Rückluftkanals **17** in die Umluftzone **15,** nämlich in der in die Umluftzone **15** hineinragenden Auskragung **171,** ein Rückluftfilter **5** eingebaut. Einen solchen zweiten Rückluftkanal **17** wird man für Containments **1** konzipieren, bei denen grössere Luftvolumina zu verarbeiten sind.

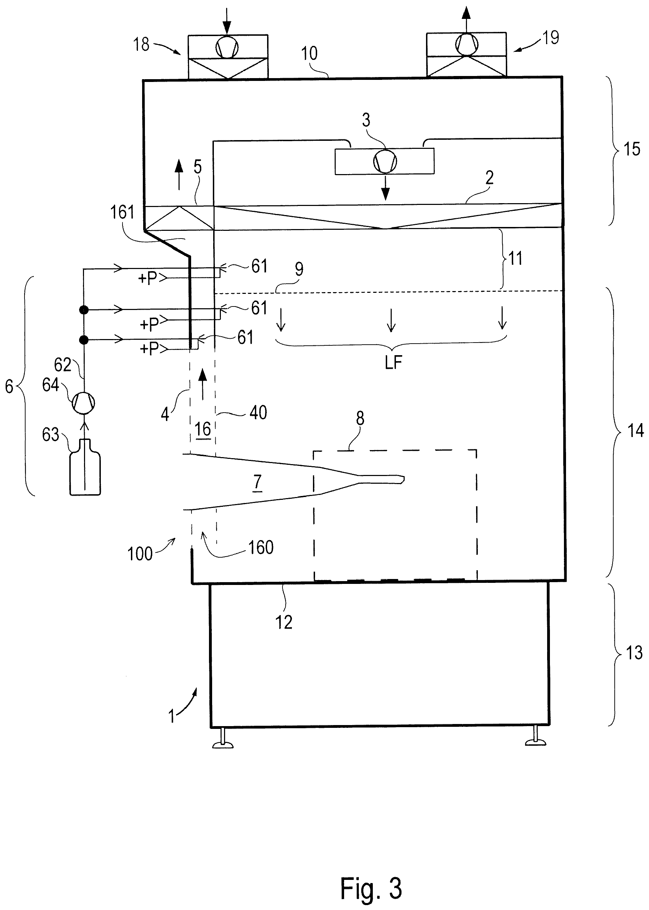

### Figur 3 - Dritte Ausführungsform

Eine Abweichung im Vergleich zur ersten Ausführungsform gemäss Figur 1 betrifft die Gestaltung der Mündung des Rückluftkanals **16** in die Umluftzone **15**, jetzt nämlich als die Umluftzone **15** erweiternde Auskragung **161**, in welcher der Rückluftfilter **5** installiert ist, wodurch sich der Umluftfilter **2** strömungstechnisch vorteilhaft nun über die gesamte Breite der Arbeitskammer **14** erstrecken kann.

Ferner beruht die Dekontaminationsanordnung **6** nun auf Vernebelungsdüsen **61** mittels denen das über eine Stoffleitung **62** und eine Pumpe **64** herangeförderte Dekontaminationsmittel und Zuführung von Druckluft **+P** als Aerosol zerstäubt wird. Je nach Konzeption des Containments 1 kann zumindest eine in die Arbeitskammer **14** gerichtete Vernebelungsdüse **61** am wichtigsten sein. Bei grösseren Arbeitskammern **14** kann es erforderlich werden, mehrere in die Arbeitskammer **14** mündende Vernebelungsdüsen **61** vorzusehen, die man dann vorteilhaft so zueinander versetzt anordnet, dass das als Dekontaminationsmittel eingebrachte Aerosol in der Arbeitskammer **14** zirkuliert. Beim Betrieb des Containments **1** im Dekontaminationsmodus mit den Vernebelungsdüsen **61** ist der Umluftventilator 3 entweder ganz abgeschaltet oder soweit heruntergeregelt, dass kein Volumenstrom gefördert wird. Je nach Grösse und Verwendung des Containments 1 kann man ergänzend im Zwischenraum **11** und/oder im ersten Rückluftkanal **16** je eine weitere Vernebelungsdüse **61** mit zuführender Stoffleitung **62** und Druckluft **+P** positionieren.

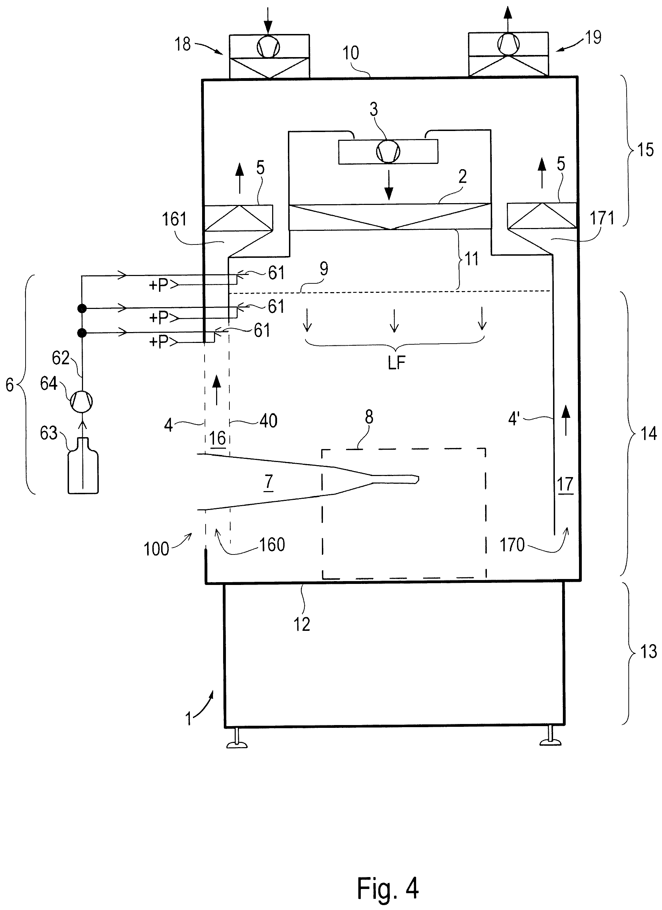

### Figur 4 - Vierte Ausführungsform

Hier ist eine Mischform des Aufbaus des Containments **1** illustriert. Aus der zweiten Ausführungsform gemäss Figur 2 stammen die beiden Rückluftkanäle **16**,**17** mit der jeweiligen Einströmöffnung **160**,**170** und den beiden in die Umluftzone **15** hineinragenden Auskragungen **161**,**171** mit dem jeweils darin eingebauten Rückluftfilter **5.** Aus der dritten Ausführungsform gemäss Figur 3 stammt die Dekontaminationsanordnung **6** mit den Vernebelungsdüsen **61**, der Stoffleitung **62** und der Zufuhr von Druckluft **+P**.

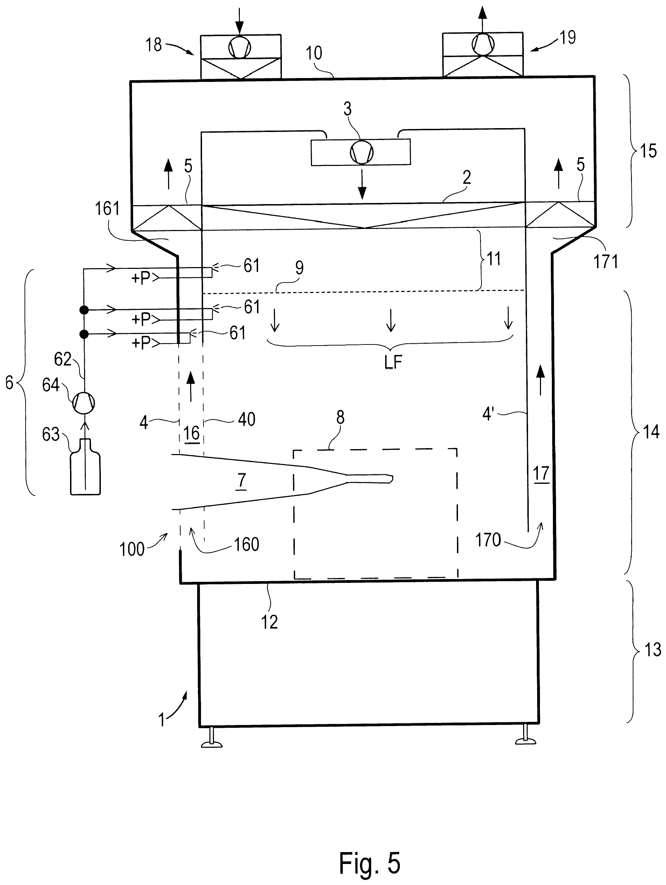

### Figur 5 - Fünfte Ausführungsform

Gegenüber dem vorherigen Aufbau gemäss Figur 4 ist jetzt, wie im Prinzip nach Konzept gemäss Figur 3, die jeweilige Mündung beider Rückluftkanäle **16**,**17** in die Umluftzone **15** als die Umluftzone **15** erweiternde Auskragung **161**,**171** gestaltet, in denen jeweils ein Rückluftfilter **5** installiert ist. Damit lässt sich der Umluftfilter **2** wieder strömungstechnisch optimal über die gesamte Breite der Arbeitskammer **14** anordnen.

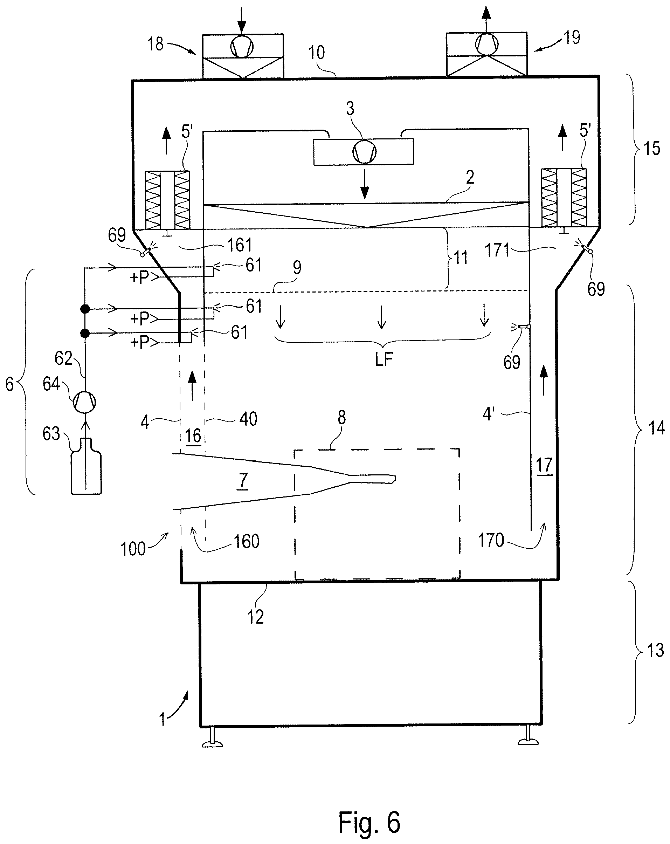

### Figur 6 - Sechste Ausführungsform

Dieser Aufbau ist eine Modifikation der Konstruktion gemäss Figur 5, anstelle der bisher in der jeweiligen erweiternden Auskragung **161**,**171** eingebauten Rückluftfilter **5** in Gestalt von Plattenfiltern, kommen nun auswechselbare Filterpatronen **5'** zum Einsatz. Ausserdem mündet in jede der Auskragungen **161**,**171**, jeweils unterhalb der Filterpatrone **5'**, eine Waschdüse **69**. Zusätzlich ist zumindest eine Waschdüse **69** in die Arbeitskammer **14** gerichtet.

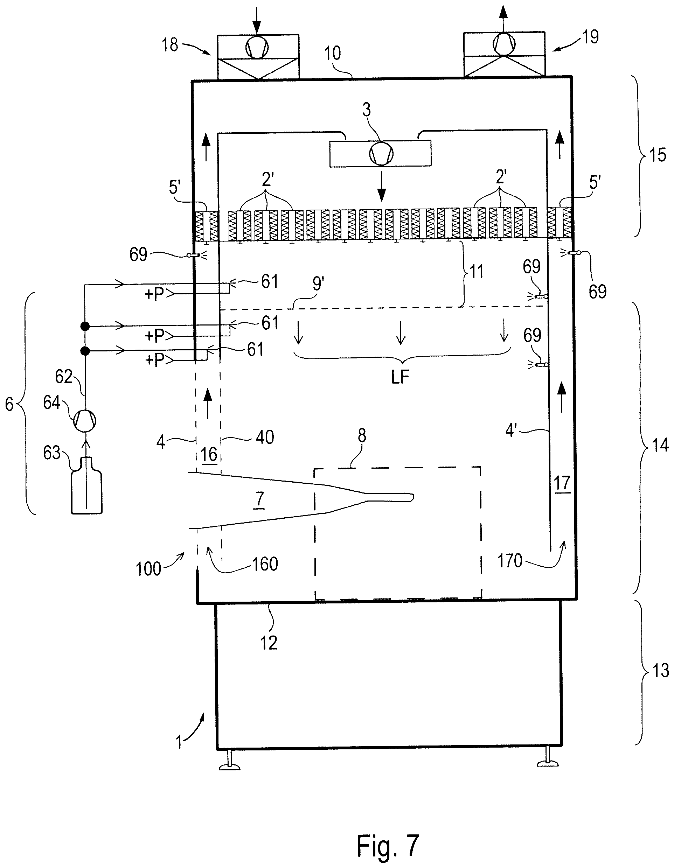

### Figur 7 - Siebente Ausführungsform

Bei diesem Konzept sind wiederum die beiden Rückluftkanäle **16**,**17** vorhanden, die jedoch ohne Auskragungen, quasi mit linearem Querschnitt in die Umluftzone **15** übergehen. In jedem der Rückluftkanäle **16**,**17**, zum Übergang in die Umluftzone **15** rückversetzt, ist eine auswechselbaren Filterpatrone als Rückluftfilter **5'** eingebaut. Der Umluftfilter **2'** besteht nun aus einer in Serie angeordneten auswechselbaren Filterpatronen. Wiederum jeweils unterhalb der Filterpatrone **5'**, also entgegen der Strömungsrichtung, ist eine Waschdüse **69** in den betreffenden Rückluftkanal **16**,**17** gerichtet. Zusätzlich zur Ausführungsform gemäss Figur 6 ist nun auch zumindest eine in den Zwischenraum **11** gerichtete Waschdüse **69** installiert. In diesem Fall wird aufgrund der festeren Haltbarkeit gegenüber der Waschflüssigkeit anstelle des bisher verwendeten Luftleitelements in Gestalt eines CG-Verteilers, jetzt ein Luftleitelement **9'** in Gestalt eines Lochblechs verwendet. Für das Handling vorteilhaft sind die in die Rückluftkanäle **16**,**17** bzw. in den Zwischenraum **11** gerichteten Waschdüsen **69** fest installiert, während die in die Arbeitskammer **14** gerichtete Waschdüse **69** vorzugsweise an einer Handpistole sitzt. Die gesamte Dekontaminationsanordnung **6** ist zu den Ausführungen gemäss den Figuren 3 bis 6 identisch.

## Patentansprüche

1. Aufbau eines Containments (**1**) mit einer aseptischen Arbeitskammer (**11**) und einer zugeordneten Dekontaminationsanordnung (6), wobei:
a) die Arbeitskammer (**14**) unten einen Boden (**12**) aufweist;
b) sich oberhalb der Arbeitskammer (**14**) eine Umluftzone (**15**) befindet, in der ein Umluftfilter (**2**,**2**') mit einem Umluftventilator (**3**) angeordnet ist;
c) Umluftzone (**15**) und Arbeitskammer (**14**) durch ein Gehäuse (**10**) nach aussen abgegrenzt sind;
d) aus der Arbeitskammer (**14**) zumindest ein erster Rückluftkanal (16) in die Umluftzone (**15**) führt; und
e) ein Rückluftfilter (**5**,**5**') strömungstechnisch mit dem Rückluftkanal (**16**) verbunden ist, **dadurch gekennzeichnet, dass**
f) der Rückluftfilter (**5**,**5**') der Umluftzone (**15**) zugewandt angeordnet ist.

2. Aufbau nach Anspruch 1, **dadurch gekennzeichnet, dass** der Rückluftfilter (**5**,**5**') an der Mündung des Rückluftkanals (**16**) in die Umluftzone (**15**) oder im Rückluftkanal (**16**) angeordnet ist.

3. Aufbau nach Anspruch 2, **dadurch gekennzeichnet, dass** die den Rückluftfilter (**5,5**') aufnehmende Mündung des Rückluftkanals (**16**):
a) als die Umluftzone (**15**) erweiternde oder in die Umluftzone (**15**) hineinragende Auskragung (**161**) ausgebildet ist; oder
b) ohne Auskragung, quasi mit linearem Querschnitt, in die Umluftzone (**15**) übergeht.

4. Aufbau nach zumindest einem der Ansprüche 1 bis 3, **dadurch gekennzeichnet, dass**
a) der Rückluftfilter (**5,5'**) die Gestalt eines Plattenfilters oder einer auswechselbaren Filterpatrone (**5'**) oder eines ersetzbaren Filtereinsatzes hat; und
b) der Umluftfilter (**2**,**2'**) die Gestalt eines Plattenfilters oder einer auswechselbaren Filterpatrone (**2'**) oder eines ersetzbaren Filtereinsatzes hat.

5. Aufbau nach zumindest einem der Ansprüche 1 bis 4, **dadurch gekennzeichnet, dass**:
a) ein einzelner Rückluftfilter (**5**,**5**') oder eine Serie von zusammenwirkenden Rückluftfiltern (**5**,**5**') vorgesehen ist; und
b) ein einzelner Umluftfilter (**2**,**2**') oder eine Serie von zusammenwirkenden Umluftfiltern (**2**,**2**') installiert ist.

6. Aufbau nach zumindest einem der Ansprüche 1 bis 5, **dadurch gekennzeichnet, dass** der zumindest erste Rückluftkanal (**16**):
a) eine transparente Frontscheibe (**4**) und eine dazu beabstandet angeordnete transparente Kanalscheibe (**40**) als Bestandteile aufweist; und
b) eine Einströmöffnung (**160**) besitzt, die sich oberhalb und nahe des Bodens (**12**) befindet.

7. Aufbau nach zumindest einem der Ansprüche 1 bis 6, **dadurch gekennzeichnet, dass** sich zwischen der Umluftzone (**15**) und der Arbeitskammer (**14**) ein Luftleitelement (**9**,**9**') erstreckt, das zur Erzeugung einer in der Arbeitskammer (**14**) abwärts fliessenden, gleichgerichteten Verdrängungsströmung (LF) dient.

8. Aufbau nach zumindest einem der Ansprüche 1 bis 7, **dadurch gekennzeichnet, dass** die Dekontaminationsanordnung (**6**) aufweist:
a) einen in der Umluftzone (**15**) positionierten Verdampfer (**60**), dem über eine Stoffleitung (**62**) Dekontaminationsmittel zugeführt wird; oder
b) zumindest eine Vernebelungsdüse (**61**), welcher über eine Stoffleitung (**62**) Dekontaminationsmittel zugeführt wird; wobei die zumindest eine Vernebelungsdüse (**61**) gerichtet ist in:
c) die Arbeitskammer (**14**); und/oder
d) einen Zwischenraum (**11**), der sich zwischen der Umluftzone (**15**) und dem Luftleitelement (**9**,**9**') befindet; und/oder
e) den zumindest ersten Rückluftkanal (**16**).

9. Aufbau nach Anspruch 8, **dadurch gekennzeichnet, dass** beim Betrieb des Containments (**1**) im Dekontaminationsmodus mit dem Verdampfer (**60**) in der Dekontaminationsanordnung (**6**) der Umluftventilator (**3**) mit verminderter Leistung, z.B. 50%, gesteuert ist.

10. Aufbau nach Anspruch 8, **dadurch gekennzeichnet, dass**:
a) eine Serie von in die Arbeitskammer (**14**) gerichteten Vernebelungsdüsen (**61**) vorgesehen ist; und
b) beim Betrieb des Containments (**1**) im Dekontaminationsmodus mit den Vernebelungsdüsen (**61**) in der Dekontaminationsanordnung (**6**) der Umluftventilator (**3**) zumindest soweit heruntergeregelt ist, dass kein Volumenstrom gefördert wird; wobei
c) die Vernebelungsdüsen (**61**) zueinander so versetzt angeordnet sind, dass das als Dekontaminationsmittel eingebrachte Aerosol in der Arbeitskammer (**14**) zirkuliert.

11. Aufbau nach zumindest einem der Ansprüche 8 bis 10, **dadurch gekennzeichnet, dass**:
a) das über die Stoffleitung (**62**) dem in der Umluftzone (**15**) positionierten Verdampfer (**60**) zugeführte Dekontaminationsmittel mittels einer Pumpe (**64**) gefördert wird; oder
b) das über die Stoffleitung (**62**) der zumindest einen Vernebelungsdüse (**61**) zugeführte Dekontaminationsmittel von einer Pumpe (**64**) gefördert und mittels Druckluft (**+P**) als Aerosol zerstäubt wird.

12. Aufbau nach zumindest einem der Ansprüche 1 bis 11, **dadurch gekennzeichnet, dass** eine Waschdüse (**69**) gerichtet ist in:
a) die Arbeitskammer (**14**); und/oder
b) einen Zwischenraum (**11**), der sich zwischen der Umluftzone (**15**) und dem Luftleitelement (**9**,**9'**) befindet; und/oder
c) den zumindest ersten Rückluftkanal (**16**), nämlich entgegen der Strömungsrichtung vor dem Rückluftfilter (**5**,**5'**).

13. Aufbau nach Anspruch **12**, **dadurch gekennzeichnet, dass**
a) die in den Rückluftkanal (**16**) und/oder in den Zwischenraum (**11**) gerichteten Waschdüsen (**69**) fest installiert sind; während
b) die in die Arbeitskammer (**14**) gerichtete Waschdüse (**69**) fest installiert ist oder an einer Handpistole sitzt.

14. Aufbau nach zumindest einem der Ansprüche 1 bis **13**, **dadurch gekennzeichnet, dass** zusätzlich ein zweiter Rückluftkanal (**17**) vorgesehen ist, wobei:
a) der zweite Rückluftkanal (**17**) einen Wandabschnitt des Gehäuses (**10**) und eine dazu beabstandete Kanalwandung (**4'**) aufweist und eine Einströmöffnung (**170**) besitzt, die sich oberhalb und nahe des Bodens (**12**) befindet;
b) der Rückluftfilter (**5**,**5'**) der Umluftzone (**15**) zugewandt, nämlich an der Mündung des zweiten Rückluftkanals (**17**) in die Umluftzone (**15**) oder im zweiten Rückluftkanal (**17**) angeordnet ist;
c) die den Rückluftfilter (**5**,**5'**) aufnehmende Mündung des zweiten Rückluftkanals (**17**) als die Umluftzone (**15**) erweiternde oder in die Umluftzone (**15**) hineinragende Auskragung (**171**) ausgebildet sein kann, oder ohne Auskragung, quasi mit linearem Querschnitt, in die Umluftzone (**15**) übergeht;
d) der Rückluftfilter (**5,5'**) die Gestalt eines Plattenfilters oder einer auswechselbaren Filterpatrone (**5'**) oder eines ersetzbaren Filtereinsatzes hat;
e) der Umluftfilter (**2**,**2'**) die Gestalt eines Plattenfilters oder einer auswechselbaren Filterpatrone (**2'**) oder eines ersetzbaren Filtereinsatzes hat;
f) ein einzelner Rückluftfilter (**5**,**5'**) oder eine Serie von zusammenwirkenden Rückluftfiltern (**5**,**5'**) vorgesehen ist; und
g) ein einzelner Umluftfilter (**2**,**2'**) oder eine Serie von zusammenwirkenden Umluftfiltern (**2**,**2'**) installiert ist.

15. Aufbau nach Anspruch 14, **dadurch gekennzeichnet, dass**
a) eine Vernebelungsdüse (**61**) in den zweiten Rückluftkanal (**17**) gerichtet ist; und
b) eine Waschdüse (**69**) in den zweiten Rückluftkanal (**17**), nämlich entgegen der Strömungsrichtung vor dem Rückluftfilter (**5**,**5'**) gerichtet ist.
